# EUROPEAN PATENT SPECIFICATION

(11) **EP 1 212 409 B1**
(45) Date of publication and mention of the grant of the patent: **14.03.2007**
(21) Application number: 00954396.8
(22) Date of filing: 21.08.2000
(51) Int. Cl.: C12N 9/28, C11D 3/386

(54) **ALKALINE BACILLUS AMYLASE**
ALKALISCHE AMYLASE AUS BACILLUS
AMYLASE ALCALINE DE BACILLUS

(30) Priority: 20.08.1999 DK 115499
(43) Date of publication of application: 12.06.2002
(73) Proprietor: Novozymes A/S, 2880 Bagsvaerd (DK)
(72) Inventor: OTTRUP, Helle, 3500 Vaerlose (DK); NIELSEN, Bjarne, Ronfeldt, 2830 Virum (DK); HOECK, Lisbeth Hedegaard, 5871Frorup (DK); ANDERSEN, Jens, Toenne, 2850 Naerum (DK)
(86) International application number: PCT/DK2000/000462
(87) International publication number: WO 2001/014532

(56) References cited:
- WO-A-99/42567
- DE-A- 2 044 513
- DATABASE WPI Section Ch, Week 199808 Derwent Publications Ltd., London, GB; Class A97, AN 1998-079853 XP002901432 & JP 09 206073 A (KAO CORP), 12 August 1997 (1997-08-12)
- MEDDA A ET AL: "New strains of Bacillus Licheniformis and Bacillus coagulans producing Thermostable alpha-Amylase Active at Alkaline pH" JOURNAL OF APPLIED BACTERIOLOGY, vol. 48, 1980, pages 47-58, XP002901433
- TAKAYA HAYASHI ET AL: "Properties of new alkaline maltohexaose-forming amylases" APPL MICROBIOL BIOTECHNOL, vol. 28, 1988, pages 281-285, XP002901434
- DATABASE BIOSIS BIOSCIENCES INFORMATION SERVICE, PHILADELPHIA, PA, US; Dialog Info Serv, file 5, BIOSIS, BAE M ET AL: "Molecular cloning and expression of alkaline amylase gene of alkalophilic bacillus-sp in bacillus-subtilis and escherichia-coli" retrieved from BIOSIS, accession no. 7114677 Database accession no. 88037422 XP002901435 & KOREAN J APPL MICROBIOL BIOENG, vol. 17, no. 2, 1989, pages 160-164,
- MC TIGUE M A ET AL: "Production studies on the alkaline amylases of three alkalophilic BACILLUS SPP." BIOTECHNOLOGY LETTERS, vol. 16, no. 6, June 1994 (1994-06), pages 569-574, XP002901436
- DATABASE BIOSIS PREV Dialog Information Services, file 5, BIOSIS PREV, TIAN X ET AL: "Studies on alkalineamylase from alkalophilic bacterium" retrieved from BIOSIS, accession no. 9527582 Database accession no. 94032582 XP002901437 & ACTA MICROBIOL SIN, vol. 31, no. 5, 1991, pages 364-370,
- DATABASE GENESEQ P:W48263 2 July 1998 (1998-07-02) PROCTER & GAMBLE CO: "Use of specific alpha-amylase enzymes - in laundry detergent compositions to provide effective cleaning and whitening of dingy fabrics" retrieved from GENESEQ P:W82263, accession no. W48263 XP002901438 & WO 98 05748 A 12 February 1998 (1998-02-12)

### SEQUENCE LISTING

<110> Novo Nordisk A/S
<120> Alkaline Bacillus Amylase
<130> 5948.000-DK
<160> 2
<170> FastSEQ for Windows Version 3.0
<210> 1
   <211> 1764
   <212> DNA
   <213> Bacillus sp.
<220>
   <221> CDS
   <222> (1)...(1764)
<221> sig_peptide
   <222> (1)...(93)
<221> mat_peptide
   <222> (94)...(1764)
<400> 1
   atg caa aac aca gcg aaa aac tcc atc tgg cag agg gtg cgc cac agc
   48
   Met Gln Asn Thr Ala Lys Asn Ser Ile Trp Gln Arg Val Arg His Ser
       -30                 -25                 -20
   gcc att gcc tta tcc gct ctc agt tta tcc ttt ggc ctg cag gcc agc
   96
   Ala Ile Ala Leu Ser Ala Leu Ser Leu Ser Phe Gly Leu Gln Ala Ser
   -15                 -10                 -5                   1
   gag tta cca caa att cca cca cag cag gtg aac aac acc atg tac cag      144
   Glu Leu Pro Gln Ile Pro Pro Gln Gln Val Asn Asn Thr Met Tyr Gln
               5                   10                  15
   gca ttt tat tgg gat gcc tac cct ggc ctt tgg gcc aat tta ccg gcc      192
   Ala Phe Tyr Trp Asp Ala Tyr Pro Gly Leu Trp Ala Asn Leu Pro Ala
           20                  25                  30
   atg gcg gcc cct ttg gcc gag cgt ggc att acc tcg atg tgg ttg ccg      240
   Met Ala Ala Pro Leu Ala Glu Arg Gly Ile Thr Ser Met Trp Leu Pro
       35                  40                  45
   ccc gcc gcc aaa ggc atg aat ggt act ttc agt gtc ggt tac gat gta      288
   Pro Ala Ala Lys Gly Met Asn Gly Thr Phe Ser Val Gly Tyr Asp Val
   50                  55                  60                  65
   tac gat ttc tgg gat ctg ggc gag ttt aac caa aaa ggc acc acc gcc      336
   Tyr Asp Phe Trp Asp Leu Gly Glu Phe Asn Gln Lys Gly Thr Thr Ala
                   70                  75                  80
   acc cgt tac ggt act cgt cag cag ctg caa caa gca ctg agt gct ctg      384
   Thr Arg Tyr Gly Thr Arg Gln Gln Leu Gln Gln Ala Leu Ser Ala Leu
               85                  90                  95
   gac caa ctg ggt att cag gcc tat ttt gat gtg gtg ttt aac cac cgc      432
   Asp Gln Leu Gly Ile Gln Ala Tyr Phe Asp Val Val Phe Asn His Arg
   100 105 110
   atg ggc gcc gat gca cag gag aat att cct ggc ttt ggc ctg gcc tgg
   480
   Met Gly Ala Asp Ala Gln Glu Asn Ile Pro Gly Phe Gly Leu Ala Trp
   115 120 125
   acc gag tat cat ctg caa ggt cgt cag gcg cat tat acc cag caa aac
   528
   Thr Glu Tyr His Leu Gln Gly Arg Gln Ala His Tyr Thr Gln Gln Asn
   130 135 140 145
   tgg ggc tac ttg tgg cac gac ttt gac tgg aac tgg acc gcg ttt aat
   576
   Trp Gly Tyr Leu Trp His Asp Phe Asp Trp Asn Trp Thr Ala Phe Asn
   150 155 160
   ggc tcc gac aat cag ctc tac ccc ggc aaa tgg tgg ggc aat acc ttc
   624
   Gly Ser Asp Asn Gln Leu Tyr Pro Gly Lys Trp Trp Gly Asn Thr Phe
   165 170 175
   cac ttc cct tat ttg atg ggt gag gat gtc gat tac aac cgc ttt gaa
   672
   His Phe Pro Tyr Leu Met Gly Glu Asp Val Asp Tyr Asn Arg Phe Glu
   180 185 190
   gtg cag cag gaa atg aaa gcc tgg ggc gag tgg atc atc aac agc gtt
   720
   Val Gln Gln Glu Met Lys Ala Trp Gly Glu Trp Ile Ile Asn Ser Val
   195 200 205
   ggc ttt agc ggc ttt cgg atg gat gcc atc gcc cat gtc gat acc gat
   768
   Gly Phe Ser Gly Phe Arg Met Asp Ala Ile Ala His Val Asp Thr Asp
   210 215 220 225
   ttt acc cgt gac tgg atc aat cac gtg cag tgg gcc acc agt gag gat 816
   Phe Thr Arg Asp Trp Ile Asn His Val Gln Trp Ala Thr Ser Glu Asp
   230 235 240
   gtg ttc ttt gtc gct gaa gcc tgg gtc agt gat atc aac ggc tat ctg 864
   Val Phe Phe Val Ala Glu Ala Trp Val Ser Asp Ile Asn Gly Tyr Leu
   245 250 255
   gat gca gtc aat acg ccg cat ttg cgc gct ttt gat ttc aat ttg cgc 912
   Asp Ala Val Asn Thr Pro His Leu Arg Ala Phe Asp Phe Asn Leu Arg
   260 265 270
   gaa gac ttc gtt gct tta agc agc ggt agc aaa gac atg cgt tgg tgg 960
   Glu Asp Phe Val Ala Leu Ser Ser Gly Ser Lys Asp Met Arg Trp Trp
   275 280 285
   ggc ggt ctg gtc aat agc cag cac cgt gat cgg gcg gtc act ttt gtc 1008
   Gly Gly Leu Val Asn Ser Gln His Arg Asp Arg Ala Val Thr Phe Val
   290 295 300 305
   gat aac cac gat acc agc cgg gcc ggc aac cct tat ggc atg ccg cag 1056
   Asp Asn His Asp Thr Ser Arg Ala Gly Asn Pro Tyr Gly Met Pro Gln
   310 315 320
   gtg atc aac tac aag aac cag gcc tac gct tac att ctg ttg cgt gag 1104
   Val Ile Asn Tyr Lys Asn Gln Ala Tyr Ala Tyr Ile Leu Leu Arg Glu
   325 330 335
   cat ggg gtg ccg act gtg ttt gcc cgc gat tac gac gaa ttt ggc atg 1152
   His Gly Val Pro Thr Val Phe Ala Arg Asp Tyr Asp Glu Phe Gly Met
   340 345 350
   gcg cca acg ctg gat aaa ttg att gag gcg cgc cgc tac ttt gct tat 1200
   Ala Pro Thr Leu Asp Lys Leu Ile Glu Ala Arg Arg Tyr Phe Ala Tyr
   355 360 365
   ggt cct ggc cat gag tac tcc ggc aat acc gag gcc gtc tac gcc tat 1248
   Gly Pro Gly His Glu Tyr Ser Gly Asn Thr Glu Ala Val Tyr Ala Tyr
   370 375 380 385
   gtg cgc gaa ggg ctt agc act gtg ccg ggt acc ggt ctg gtg atg ctg 1296
   Val Arg Glu Gly Leu Ser Thr Val Pro Gly Thr Gly Leu Val Met Leu
   390 395 400
   ata tcg ggt cga aac tgg ggt ggt cag cag tcg ttc acc atc aac agc 1344
   Ile Ser Gly Arg Asn Trp Gly Gly Gln Gln Ser Phe Thr Ile Asn Ser
   405 410 415
   cac cag ccg aat acc acc ttt tac gat tat acc ggc aat gtt agc ggc 1392
   His Gln Pro Asn Thr Thr Phe Tyr Asp Tyr Thr Gly Asn Val Ser Gly
   420 425 430
   acg gtg acc acc aat gcg cag ggc tat ggc agc ttc ccg gtc act atg 1440
   Thr Val Thr Thr Asn Ala Gln Gly Tyr Gly Ser Phe Pro Val Thr Met
   435 440 445
           acg gaa agt acc ggt tgg tca gtc tgg gta cca caa tcc aat ggt ggc
   1488
           Thr Glu Ser Thr Gly Trp Ser Val Trp Val Pro Gln Ser Asn Gly Gly
           450                 455                 460                 465
           act cag ccg gga tcc att acc ctg cgg atg acc aag gat gtt ggc tat
   1536
           Thr Gln Pro Gly Ser Ile Thr Leu Arg Met Thr Lys Asp Val Gly Tyr
                           470                 475                 480
           ggc ttt tcg ttg ttc ttc acc ggc agc agt gcg gaa ctg acc aac tgg
   1584
           Gly Phe Ser Leu Phe Phe Thr Gly Ser Ser Ala Glu Leu Thr Asn Trp
                       485                 490                 495
           ggc ggc ggt att gaa ggc acc tgg aca tcc ggt aat gtc tgg gaa gtg
   1632
           Gly Gly Gly Ile Glu Gly Thr Trp Thr Ser Gly Asn Val Trp Glu Val
                   500                 505                 510
           acc atc ccg gat ccg ggc aac ttt gaa tgg aaa acc cgt aaa ggc cca
   1680
           Thr Ile Pro Asp Pro Gly Asn Phe Glu Trp Lys Thr Arg Lys Gly Pro
               515                 520                 525
           acc ggt ggc agt ggt cag gac tgg gaa agt ggc agc aac cac aat cag
   1728
           Thr Gly Gly Ser Gly Gln Asp Trp Glu Ser Gly Ser Asn His Asn Gln
           530                 535                 540                 545
           acc aat ttg cac ccc agt ttt aat ggt ggg ttt taa
   1764
           Thr Asn Leu His Pro Ser Phe Asn Gly Gly Phe  *
                           550                 555
<210> 2
   <211> 587
   <212> PRT
   <213> Bacillus sp.
<220>
   <221> SIGNAL
   <222> (1)...(31)
<400> 2
   Met Gln Asn Thr Ala Lys Asn Ser Ile Trp Gln Arg Val Arg His Ser
       -30                 -25                 -20
   Ala Ile Ala Leu Ser Ala Leu Ser Leu Ser Phe Gly Leu Gln Ala Ser
   -15                 -10                 -5                   1
   Glu Leu Pro Gln Ile Pro Pro Gln Gln Val Asn Asn Thr Met Tyr Gln
               5                   10                  15
   Ala Phe Tyr Trp Asp Ala Tyr Pro Gly Leu Trp Ala Asn Leu Pro Ala
           20                  25                  30
   Met Ala Ala Pro Leu Ala Glu Arg Gly Ile Thr Ser Met Trp Leu Pro
       35                  40                  45
   Pro Ala Ala Lys Gly Met Asn Gly Thr Phe Ser Val Gly Tyr Asp Val
   50                  55                  60                  65
   Tyr Asp Phe Trp Asp Leu Gly Glu Phe Asn Gln Lys Gly Thr Thr Ala
                   70                  75                  80
   Thr Arg Tyr Gly Thr Arg Gln Gln Leu Gln Gln Ala Leu Ser Ala Leu
               85                  90                  95
   Asp Gln Leu Gly Ile Gln Ala Tyr Phe Asp Val Val Phe Asn His Arg
           100                 105                 110
   Met Gly Ala Asp Ala Gln Glu Asn Ile Pro Gly Phe Gly Leu Ala Trp
       115                 120                 125
   Thr Glu Tyr His Leu Gln Gly Arg Gln Ala His Tyr Thr Gln Gln Asn
   130                 135                 140                 145
   Trp Gly Tyr Leu Trp His Asp Phe Asp Trp Asn Trp Thr Ala Phe Asn
                   150                 155                 160
   Gly Ser Asp Asn Gln Leu Tyr Pro Gly Lys Trp Trp Gly Asn Thr Phe
               165                 170                 175
   His Phe Pro Tyr Leu Met Gly Glu Asp Val Asp Tyr Asn Arg Phe Glu
   180 185 190
   Val Gln Gln Glu Met Lys Ala Trp Gly Glu Trp Ile Ile Asn Ser Val
   195 200 205
   Gly Phe Ser Gly Phe Arg Met Asp Ala Ile Ala His Val Asp Thr Asp
   210 215 220 225
   Phe Thr Arg Asp Trp Ile Asn His Val Gln Trp Ala Thr Ser Glu Asp
   230 235 240
   Val Phe Phe Val Ala Glu Ala Trp Val Ser Asp Ile Asn Gly Tyr Leu
   245 250 255
   Asp Ala Val Asn Thr Pro His Leu Arg Ala Phe Asp Phe Asn Leu Arg
   260 265 270
   Glu Asp Phe Val Ala Leu Ser Ser Gly Ser Lys Asp Met Arg Trp Trp
   275 280 285
   Gly Gly Leu Val Asn Ser Gln His Arg Asp Arg Ala Val Thr Phe Val
   290 295 300 305
   Asp Asn His Asp Thr Ser Arg Ala Gly Asn Pro Tyr Gly Met Pro Gln
   310 315 320
   Val Ile Asn Tyr Lys Asn Gln Ala Tyr Ala Tyr Ile Leu Leu Arg Glu
   325 330 335
   His Gly Val Pro Thr Val Phe Ala Arg Asp Tyr Asp Glu Phe Gly Met
   340 345 350
   Ala Pro Thr Leu Asp Lys Leu Ile Glu Ala Arg Arg Tyr Phe Ala Tyr
   355 360 365
   Gly Pro Gly His Glu Tyr Ser Gly Asn Thr Glu Ala Val Tyr Ala Tyr
   370 375 380 385
   Val Arg Glu Gly Leu Ser Thr Val Pro Gly Thr Gly Leu Val Met Leu
   390 395 400
   Ile Ser Gly Arg Asn Trp Gly Gly Gln Gln Ser Phe Thr Ile Asn Ser
   405 410 415
   His Gln Pro Asn Thr Thr Phe Tyr Asp Tyr Thr Gly Asn Val Ser Gly
   420 425 430
   Thr Val Thr Thr Asn Ala Gln Gly Tyr Gly Ser Phe Pro Val Thr Met
   435 440 445
   Thr Glu Ser Thr Gly Trp Ser Val Trp Val Pro Gln Ser Asn Gly Gly
   450 455 460 465
   Thr Gln Pro Gly Ser Ile Thr Leu Arg Met Thr Lys Asp Val Gly Tyr
                   470                     475                 480
   Gly Phe Ser Leu Phe Phe Thr Gly Ser Ser Ala Glu Leu Thr Asn Trp
               485                     490                 495
   Gly Gly Gly Ile Glu Gly Thr Trp Thr Ser Gly Asn Val Trp Glu Val
           500                     505                 510
   Thr Ile Pro Asp Pro Gly Asn Phe Glu Trp Lys Thr Arg Lys Gly Pro
       515                     520                 525
   Thr Gly Gly Ser Gly Gln Asp Trp Glu Ser Gly Ser Asn His Asn Gln
   530                     535                     540                 545
   Thr Asn Leu His Pro Ser Phe Asn Gly Gly Phe
                   550                     555

## Description

### FIELD OF INVENTION

The present invention relates to amylases having improved washing performance in an alkaline detergent solution at low temperature.

### BACKGROUND OF THE INVENTION

For a number of years alpha-amylase enzymes have been used for a variety of different purposes, the most important of which are starch liquefaction, textile desizing, starch modification in the paper and pulp industry, and for brewing and baking. A further use of alpha-amylases, which is becoming increasingly important, is the removal of starchy stains during washing with a detergent at alkaline pH.

Examples of commercial alpha-amylase products are Termamyl^{®}, BAN^{®} and Fungamyl^{®}, all available from Novo Nordisk A/S, Denmark. These and similar products from other commercial sources have an acidic to a neutral pH optimum, typically in the range of from pH 5 to pH 7.5, and they do not display optimal activity in detergent solutions at alkaline pH.

WO 95/26397 discloses an alpha-amylase from a *Bacillus* strain, which has optimum activity at pH 8. WO 96/23873 describes variants of *Bacillus* amylases with improved performance under washing conditions.

US 5,147,796 describes an alkaline pullulanase having alpha-amylase activity. Fig. 2b of the document shows optimum amylase activity at pH 8-8.5.

M. Takagi et al., J. Ferment. Bioeng., vol 81, No. 6, 557-559 (1996) describe an alkaliphilic alpha-amylase-pullulanase from Bacillus sp. The enzyme has optimum amylase activity at pH 9, but the activity drops rapidly at higher pH, and the activity at pH 10 is lower than at pH 7.

It is an object of the present invention to provide novel alpha-amylases with improved performance in alkaline solutions, especially in alkaline detergent solutions at pH around 9-11.

### SUMMARY OF THE INVENTION

The present invention provides alpha-amylase, which is:
a) a polypeptide produced by *Bacillus* sp. NCIMB 40916, or
b) a polypeptide having an amino acid sequence as shown in positions 1-556 of SEQ ID NO: 2, or
c) a polypeptide encoded by the alpha-amylase encoding part of the DNA sequence cloned into a plasmid present in *Escherichia coli* DSM 13001, or
d) an analogue of the polypeptide defined in (a) or (b) which:
   i) is at least 60 % homologous with said polypeptide, or
   ii) is derived from said polypeptide by substitution, deletion and/or insertion of one with the proviso that the polypeptide does not have the amino acid sequence as shown in positions 32-532 of seq. 20 n° 2 disclosed in WO 99/42567 or an analogue thereof which is at least 60% homologous with said polypeption.

In another aspect, the invention provides an alpha-amylase having one or more of the following characteristics:
- an activity at pH 10.5 which is at least two times higher than the activity at pH 7.3 when measured at 37°C.
- an activity at pH 9.5 which is at least 4 times higher than the activity at pH 7.3 when measured at 37°C.
- an optimum pH of about 9.5 when measured at 37°C.
- a thermostability such that it retains more than 90 % of its activity after 20 minutes incubation at 25°C in a solution of 3 g/l of a test detergent containing 20% STPP, 25% Na₂SO₄, 15% Na₂CO₃, 20% LAS, 5% C12-C15 alcohol ethoxylate, 5% Na₂Si₂O₅, 0.3% NaCl at pH 10.5 and 6 degrees German hardness, and retains less than 90 % of its activity after 20 minutes incubation at 30°C in the same solution.
- a molecular weight of about 55 kDa as determined by SDS-PAGE.
- an iso-electric point of about 5 as determined by isoelectric focusing.

The invention also provides an isolated DNA sequence, which encodes an alpha-amylase, wherein the alpha-amylase is that described above, or wherein the DNA sequence comprises:
a) the DNA sequence shown in positions 94-1764 of SEQ ID NO: 1, or
b) an analogue of the DNA sequence defined in a) which
   i) is at least 60 % homologous with said DNA sequence, or
   ii) hybridizes with said DNA sequence at least 55°C.
With the proviso that the DNA sequence does not have the DNA sequence shown in positions 94-1596 of seq. ID n°1 disclosed in WO 99/42567 or an analogue thereof which is at least 60% homologous with said sequences.

Other aspects of the invention provide a recombinant expression vector comprising the DNA sequence, and a cell transformed with the DNA sequence or the recombinant expression vector.

The invention also provides a method for producing an alpha-amylase by cultivating the cell and a detergent composition comprising said alpha-amylase.

### BRIEF DESCRIPTION OF DRAWINGS

The present invention is further illustrated with reference to the accompanying drawings, in which:
Fig. 1 shows a pH activity profile of the amylase from NCIMB 40916 compared to two prior-art amylases (SP722 and Termamyl).
Fig. 2 shows a temperature activity profile of the amylase from NCIMB 40916.
Fig. 3 shows the stability of the amylase from NCIMB 40916 after incubation at various temperatures.
Fig. 4 shows the cloning vector pSJ1678 described in Example 1.
Figs. 5 and 6 show results of washing tests described in Example 4.

### DETAILED DESCRIPTION OF THE INVENTION

### Microbial source

The alpha-amylase of the invention may be derived from a strain of Bacillus. A preferred strain is Bacillus sp. NCIMB 40916. This strain was deposited on 28 January 1998 by the inventors under the terms of the Budapest Treaty on the International Recognition of the Deposit of Microorganisms for the Purposes of Patent Procedure at the National Collections of Industrial and Marine Bacteria Limited (NCIMB), 23 St. Machar Drive, Aberdeen AB2 1RY, Scotland, United Kingdom. An Escherichia coli strain termed NN049489 containing the alpha amylase gene is shown in SEQ ID NO: 1 cloned in plasmid pJA386 has also been deposited on 7 August 1999 under the terms of the Budapest Treaty with the Deutshe Sammmlung von Microorganismen und Zellkulturen GmbH (DSMZ), Mascheroder Weg 1b, D-38124 Braunschweig DE, and given the accession number DSM 13001.

### Production of alpha-amylase

The alpha-amylase of the invention can be produced by cultivating a suitable amylase-producing strain of Bacillus or the transformed host cell of the invention in a suitable nutrient medium, and recovering the alpha-amylase from the culture medium.

The medium used to cultivate the cells may be any conventional medium suitable for growing the host cell in question and obtaining expression of the alpha-amylase of the invention. Suitable media are available from commercial suppliers or may be prepared according to published recipes (e.g. as described in catalogues of the American Type Culture Collection).

The alpha-amylase secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### Properties of alpha-amylase

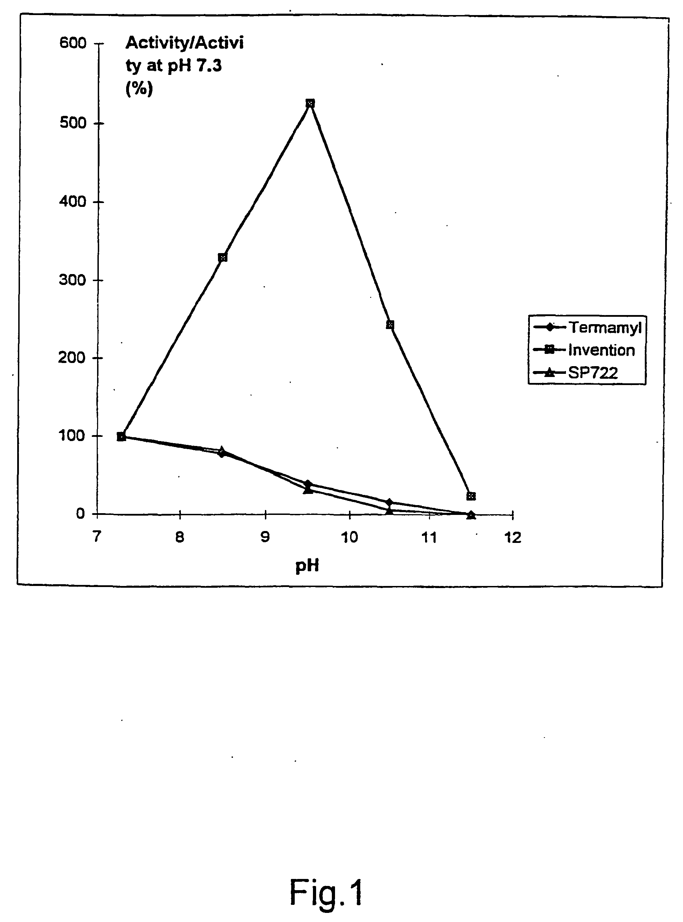

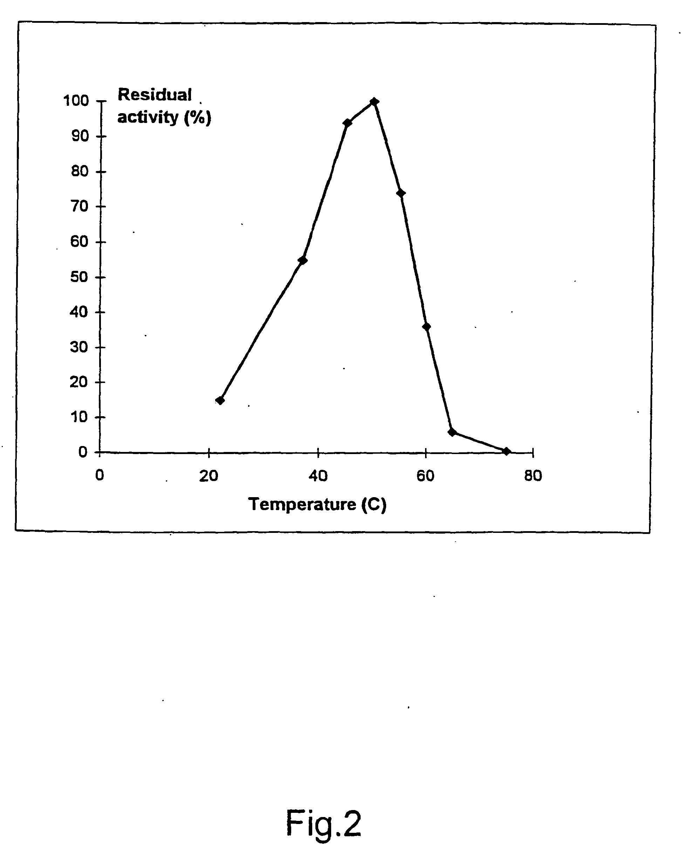

A preferred alpha-amylase is derived from Bacillus sp. NCIMB 40916. It can be produced as described in the examples. The full length amino acid sequence of the amylase and the DNA encoding it are shown in SEQ ID NO: 1 and 2. The following characteristics were found for the amylase of the invention (purified alpha-amylase from NCIMB 40916):
A molecular weight of approximately 55 kDa as determined by SDS-PAGE using a Novex, 4-25% gradient gel.
A pI of approximately 5 was determined by isoelectric focusing (Ampholine PAG, pH 3.5-9.5).
A pH-activity curve is shown in Fig. 1, taking the activity at pH 7.3 as 100%. It was determined using the Phadebas assay using 50 mM Britten-Robinson buffer adjusted to various pH-values. For reference, the pH profiles of two prior-art Bacillus amylases (Termamyl derived from B. licheniformis and SP722 produced according to WO 95/26397) were measured under the same conditions are also shown in Figure 1. Figure 1 shows that the amylase of the invention has about 10 times higher activity than the two prior-art amylases at pH 9.5. Fig. 1 also shows that the optimum activity is about pH 9.5.
A temperature-activity curve was measured using the Phadebas assay at various temperatures with 50 mM Britten-Robinson buffer adjusted to pH 9.5. The results are shown in Fig. 2. It is seen from Fig. 2 that the amylase of the invention has optimum activity at about 55°C .

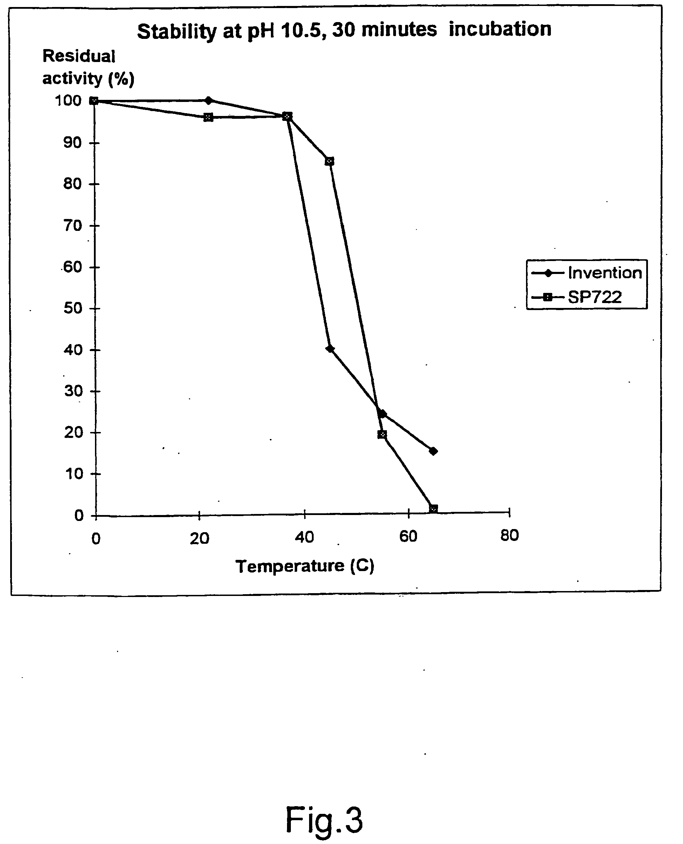

Stability of the amylase was measured at pH 10.5 (50 mM CAPS) at 22, 37, 45, 55 and 65 °C after 30 minutes incubation. The enzyme was diluted to 40 NJ/ml in buffer and 25 microlitre sample was incubated at the respective temperatures. After 30 minutes, the samples was chilled on ice and residual activity was determined. A stability profile of the amylase of the invention and a prior-art amylase (SP722) is shown in Figure 3.

Stability was tested by incubating 40 NU/ml of amylase in a solution of 3 g/l of the A/P model detergent described above, at pH 10.5 and 6°dH (German hardness, Ca:Mg 2:1). After the incubation, the residual activity was measured by Phadebas at pH 7.3. The results were 95 % residual activity after incubation at 25°C, and 87 % at 30°C.

### Homology of polypeptide and DNA sequence

The amino acid sequence homology may be determined as the degree of identity between the two sequences indicating a derivation of the first sequence from the second. The homology may suitably be determined by means of computer programs known in the art. Thus, FASTA provided in GCG version 8 (Needleman, S.B. and Wunsch, C.D., (1970), Journal of Molecular Biology, 48, 443-453) may be used with the following settings: Scoring matrix: GenRunData:blosum50.cmp, Variable pamfactor used Gap creation penalty: 12, Gap extension penalty: 2.

The amino acid sequence exhibits a degree of identity preferably of at least 60%, preferably at least 70%, more preferably at least 80%, especially at least 90% or at least 95%, even more especially at least 97% or at least 99% with the amino acid sequence shown in positions 1-556 of SEQ ID NO: 2.

The DNA sequence homology may be determined as the degree of identity between the two sequences indicating a derivation of the first sequence from the second. The homology may suitably be determined by means of computer programs known in the art such as GAP provided in the GCG program package (described above). Thus, Gap GCGv8 may be used with the following default parameters: GAP creation penalty of 5.0 and GAP extension penalty of 0.3, default scoring matrix. GAP uses the method of Needleman/Wunsch/Sellers to make alignments.

The DNA construct of the present invention comprises a DNA sequence exhibiting a degree of identity preferably of at least 60%, preferably at least 70%, more preferably at least 80%, especially at least 90% or at least 95%, even more especially at least 97% or at least 99% with the nucleic acid sequence shown in positions 94-1764 of SEQ ID NO: 1.

### Hybridization

The hybridization is used to indicate that a given DNA. sequence is analogous to a nucleotide probe corresponding to SEQ ID NO: 1. The hybridization conditions are described in detail below.

Suitable conditions for determining hybridization between a nucleotide probe and a homologous DNA or RNA sequence involves presoaking of the filter containing the DNA fragments or RNA to hybridize in 5 x SSC (standard saline citrate) for 10 min, and prehybridization of the filter in a solution of 5 x SSC (Sambrook et al. 1989), 5 x Denhardt's solution (Sambrook et al. 1989), 0.5 % SDS and 100 micro g/ml of denatured sonicated salmon sperm DNA (Sambrook et al. 1989), followed by hybridization in the same solution containing a random-primed (Feinberg, A. P. and Vogelstein, B. (1983) Anal. Biochem. 132:6-13), ³²P-dCTP-labeled (specific activity > 1 x 10⁹ cpm/micro g) probe for 12 hours at approx. 45°C. The filter is then washed two times for 30 minutes in 2 x SSC, 0.5 % SDS at a temperature of at least 55°C, more preferably at least 60°C, more preferably at least 65°C, even more preferably at least 70°C, especially at least 75°C.

Molecules to which the oligonucleotide probe hybridizes under these conditions are detected using an x-ray film.

### Recombinant expression vector

The expression vector of the invention typically includes control sequences encoding a promoter, operator, ribosome binding site, translation initiation signal, and, optionally, a repressor gene or various activator genes.

The recombinant expression vector carrying the DNA sequence encoding the alpha-amylase of the invention may be any vector, which may conveniently be subjected to recombinant DNA procedures, and the choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid, a bacteriophage or an extrachromosomal element, minichromosome or an artificial chromosome. Alternatively, the vector may be one which, when introduced into a host cell, is integrated into the host cell genome and replicated together with the chromosome(s) into which it has been integrated.

In the vector, the DNA sequence should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the DNA sequence encoding an alpha-amylase of the invention, especially in a bacterial host, are the promoter of the lac operon of *E.coli,* the *Streptomyces coelicolor agarase* gene dagA promoters, the promoters of the *Bacillus licheniformis* alpha-amylase gene (amyL), the promoters of the *Bacillus stearothermophilus* maltogenic amylase gene (amyM), the promoters of the *Bacillus amyloliquefaciens* alpha-amylase (amyQ), the promoters of the *Bacillus subtilis* xylA and xylB genes etc. For transcription in a fungal host, examples of useful promoters are those derived from the gene encoding *A. oryzae* TAKA amylase, *Rhizomucor miehei aspartic proteinase, A. niger* neutral alpha-amylase, A. niger acid stable alpha-amylase, *A. niger* glucoamylase, *Rhizomucor miehei* lipase, *A. oryzae* alkaline protease, *A. oryzae* triose phosphate isomerase or *A. nidulans* acetamidase.

The expression vector of the invention may also comprise a suitable transcription terminator and, in eukaryotes, polyadenylation sequences operably connected to the DNA sequence encoding the alpha-amylase of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194., pAMBl and pIJ702.

The vector may also comprise a selectable marker, e.g. a gene the product of which complements a defect in the host cell, such as the dal genes from *B. subtilis* or *B. licheniformis*, or one which confers antibiotic resistance such as ampicillin, kanamycin, chloramphenicol or tetracyclin resistance. Furthermore, the vector may comprise Aspergillus selection markers such as amdS, argB, niaD and sC, a marker giving rise to hygromycin resistance, or the selection may be accomplished by co-transformation, e.g., as described in WO 91/17243.

While intracellular expression may be advantageous in some respects, e.g., when using certain bacteria as host cells, it is generally preferred that the expression is extracellular.

Procedures suitable for constructing vectors of the invention encoding an alpha-amylase, and containing the promoter, terminator and other elements, respectively, are well known to persons skilled in the art [cf., for instance, Sambrook et al. (1989)].

### Host cells

The cell of the invention, either comprising a DNA construct or an expression vector of the invention as defined above, is advantageously used as a host cell in the recombinant production of the alpha-amylase of the invention. The cell may be transformed with the DNA construct of the invention encoding the amylase, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, e.g. by homologous or heterologous recombination. Alternatively, the cell may be transformed with an expression vector as described above in connection with the different types of host cells.

The cell of the invention may be a cell of a higher organism such as a mammal or an insect, but is preferably a microbial cell, e.g., a bacterial or a fungal (including yeast) cell.

Examples of bacterial host cells which, on cultivation, are capable of producing the enzyme of the invention are gram-positive bacteria such as strains of *Bacillus,* such as strains of *B. subtilis, B. licheniformis, B. lentus, B.* clausii, *B. brevis, B. stearothermophilus,* B. alkalophilus, *B. amyloliquefaciens, B. coagulans, B. circulans, B. lautus, B.* megaterium or *B. thuringiensis,* or strains of *Streptomyces,* such as *S. lividans* or *S. murinus*, or gram-negative bacteria such as *Escherichia coli*. The transformation of the bacteria may be effected by protoplast transformation, electroporation, conjugation, or by using competent cells in a manner known per se (cf. Sambrook et al., supra).

The yeast organism may favourably be selected from a species of Saccharomyces or Schizosaccharomyces, e.g. Saccharomyces cerevisiae. The filamentous fungus may advantageously belong to a species of *Aspergillus,* e.g., *Aspergillus oryzae* or *Aspergillus niger.* Fungal cells may be transformed by a process involving protoplast formation and transformation of the protoplasts followed by regeneration of the cell wall in a manner known per se. A suitable procedure for transformation of *Aspergillus* host cells is described in EP 238 023.

### Industrial Applications

Owing to their activity at alkaline pH values, the alpha-amylases of the invention are well suited for use in a variety of industrial processes, in particular the enzyme finds potential applications as a component in detergents, e.g. laundry and hard surface cleaning detergent compositions, but it may also be useful in the production of sweeteners and ethanol from starch. Thus, it may be used in conventional starch-converting processes, such as liquefaction and saccharification processes described in US Patent No. 3,912,590 and EP patent publications Nos. 252,730 and 63,909.

The alkaline alpha-amylase of the invention may also be used in the production of lignocellulosic materials, such as pulp, paper and cardboard, from starch reinforced waste paper and cardboard, especially where repulping occurs at pH above 7 and where amylases can facilitate the disintegration of the waste material through degradation of the reinforcing starch. The alpha-amylase of the invention is especially useful in a process for producing a papermaking pulp from starch-coated printed-paper. The process may be performed as described in WO 95/14807, comprising the following steps:
a) disintegrating the paper to produce a pulp,
b) treating with a starch-degrading enzyme before, during or after step a), and
c) separating ink particles from the pulp after steps a) and b).

The alpha-amylases of the invention may also be very useful in modifying starch where enzymatically modified starch is used in papermaking together with alkaline fillers such as calcium carbonate, kaolin and clays. With the alkaline alpha-amylases of the invention it becomes possible to modify the starch in the presence of the filler thus allowing for a simpler integrated process.

The alpha-amylase of the invention may also be very useful in textile desizing. In the textile processing industry, alpha-amylases are traditionally used as auxiliaries in the desizing process to facilitate the removal of starch-containing size, which has served as a protective coating on weft yarns during weaving. Complete removal of the size coating after weaving is important to ensure optimum results in the subsequent processes, in which the fabric is scoured, bleached and dyed. Enzymatic starch break-down is preferred because it does not involve any harmful effect on the fiber material. In order to reduce processing cost and increase mill throughput, the desizing processing is sometimes combined with the scouring and bleaching steps. In such cases, non-enzymatic auxiliaries such as alkali or oxidation agents are typically used to break down the starch, because traditional alpha-amylases are not very compatible with high pH levels and bleaching agents. The non-enzymatic breakdown of the starch size does lead to some fiber damage because of the rather aggressive chemicals used. Accordingly, it would be desirable to use the alpha-amylases of the invention as they have an improved performance in alkaline solutions. The alpha-amylases may be used alone or in combination with a cellulase when desizing cellulose-containing fabric or textile.

The alpha-amylases of the invention may also be very useful in a beer-making process; the alpha-amylases will typically be added during the mashing process.

### Detergent Compositions

According to the invention, the alpha-amylase may typically be a component of a detergent composition, e.g., a laundry detergent composition. As such, it may be included in the detergent composition in the form of a non-dusting granulate, a stabilized liquid, or a protected enzyme. Non-dusting granulates may be produced, e.g., as disclosed in US 4,106,991 and 4,661,452 (both to Novo Industri A/S) and may optionally be coated by methods known in the art. Examples of waxy coating materials are poly(ethylene oxide) products (polyethyleneglycol, PEG) with mean molecular weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in patent GB 1483591. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in EP 238,216.

The properties of the alpha-amylase of the invention make it particularly suitable for use in alkaline detergents, e.g., at pH 9.5-10.5, and for washing at low temperatures, e.g., 20-40°C.

The detergent composition of the invention may be in any convenient form, e.g. as powder, granules, paste or liquid. A liquid detergent may be aqueous, typically containing up to 70% water and 0-30% organic solvent, or non-aqueous.

The detergent composition comprises one or more surfactants, each of which may be anionic, nonionic, cationic, or amphoteric (zwitterionic). The detergent will usually contain 0-50% of anionic surfactant such as linear alkylbenzene-sulfonate (LAS), alpha-olefinsulfonate (AOS), alkyl sulfate (fatty alcohol sulfate) (AS), alcohol ethoxysulfate (AEOS or AES), secondary alkanesulfonates (SAS), alpha-sulfo fatty acid methyl esters, alkyl- or alkenylsuccinic acid, or soap. It may also contain 0-40% of nonionic surfactant such as alcohol ethoxylate (AEO or AE), alcohol propoxylate, carboxylated alcohol ethoxylates, nonylphenol ethoxylate, alkylpolyglycoside, alkyldimethylamine oxide, ethoxylated fatty acid monoethanolamide, fatty acid monoethanolamide, or polyhydroxy alkyl fatty acid amide (e.g. as described in WO 92/06154).

The detergent composition may additionally comprise one or more other enzymes, such as pullulanase, esterase, lipase, cutinase, protease, cellulase, peroxidase, or oxidase, e.g., laccase.

Normally the detergent contains 1-65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (e.g., SKS-6 from Hoechst).

The detergent builders may be subdivided into phosphorus-containing and non-phosphorous-containing types. Examples of phosphorus-containing inorganic alkaline detergent builders include the water-soluble salts, especially alkali metal pyrophosphates, orthophosphates, polyphosphates and phosphonates. Examples of non-phosphorus-containing inorganic builders include water-soluble alkali metal carbonates, borates and silicates as well as layered disilicates and the various types of water-insoluble crystalline or amorphous alumino silicates of which zeolites is the best known representative.

Examples of suitable organic builders include alkali metal, ammonium or substituted ammonium salts of succinates, malonates, fatty acid malonates, fatty acid sulphonates, carboxymethoxy succinates, polyacetates, carboxylates, polycarboxylates, aminopolycarboxylates and polyacetyl carboxylates. The detergent may also be unbuilt, i.e. essentially free of detergent builder.

The detergent may comprise one or more polymers. Examples are carboxymethylcellulose (CMC), poly(vinylpyrrolidone) (PVP), polyethyleneglycol (PEG), poly(vinyl alcohol) (PVA), polycarboxylates such as polyacrylates, polymaleates, maleic/acrylic acid copolymers and lauryl methacrylate/acrylic acid copolymers.

The detergent composition may contain bleaching agents of the chlorine/bromine-type or the oxygen-type. The bleaching agents may be coated or encapsulated.

Examples of inorganic chlorine/bromine-type bleaches are lithium, sodium or calcium hypochlorite or hypobromite as well as chlorinated trisodium phosphate. Examples of organic chlorine/bromine-type bleaches are heterocyclic N-bromo and N-chloro imides such as trichloroisocyanuric, tribromoisocyanuric, dibromoisocyanuric and dichloroisocyanuric acids, and salts thereof with water solubilizing cations such as potassium and sodium. Hydantoin compounds are also suitable. The bleaching system may also comprise peroxyacids of, e.g., the amide, imide, or sulfone type.

The oxygen-type bleach may be an inorganic persalt, preferably with a bleach activator, or a peroxy acid compound. Examples of inorganic persalts are alkali metal perborates, both tetrahydrates and monohydrates, alkali metal percarbonates, persilicates and perphosphates. The activator may be tetraacetylethylenediamine (TAED) or nonanoyloxybenzene-sulfonate (NOBS).

The enzymes of the detergent composition of the invention may be stabilized using conventional stabilizing agents, e.g. a polyol such as propylene glycol or glycerol, a sugar or sugar alcohol, lactic acid, boric acid, or a boric acid derivative such as, e.g., an aromatic borate ester, and the composition may be formulated as described in, e.g., WO 92/19709 and WO 92/19708. The enzymes of the invention may also be stabilized by adding reversible enzyme inhibitors, e.g., of the protein type as described in EP 0 544 777 B1.

The detergent may also contain other conventional detergent ingredients such as, e.g., fabric conditioners including clays, deflocculant material, foam boosters, foam depressors, suds suppressors, anti-corrosion agents, soil-suspending agents, anti-soil-redeposition agents, dyes, dehydrating agents, bactericides, optical brighteners, or perfume.

The pH (measured in aqueous solution at use concentration) will usually be neutral or alkaline, e.g., in the range of 7-11.

More specifically, the alpha-amylase of the invention may be incorporated in any of the detergent formulations described in WO 96/23873.

The alpha-amylases of the invention may be incorporated in concentrations conventionally employed in detergents. It is at present contemplated that, in the detergent composition of the invention, the alpha-amylase may be added in an amount corresponding to 0.00001-1 mg (calculated as pure enzyme protein) of alpha-amylase per liter of wash liquor.

The present invention is further illustrated in the following examples, which are not intended to be in any way limiting to the scope of the invention as claimed.

### EXAMPLES

### Host organism:

Escherichia coli SJ2 is described in Diderichsen, B., Wedsted, U., Hedegaard, L., Jensen, B.R., Sjoholm, C. (1990) Journal of Bacteriology, Vol.172, No. 8, p. 4315-4321.

### Plasmids:

The gene bank vector was pSJ1678, which is further disclosed in WO94/19454 which is hereby incorporated by reference.

The gene bank vector pSJ1678 is further disclosed in WO 94/19454, which is hereby incorporated by reference.

### Model detergent:

A/P (Asia/Pacific) Model Detergent has the following composition: 20% STPP (sodium tripolyphosphate), 25% Na₂SO₄, 15% Na₂CO₃, 20% LAS (linear alkylbenzene sulfonate, Nansa 80S), 5% C₁₂-C₁₅ alcohol ethoxylate (Dobanol 25-7), 5% Na₂Si₂O₅, 0.3% NaCl.

### Example 1

### Cloning of alpha-amylase into E. coli

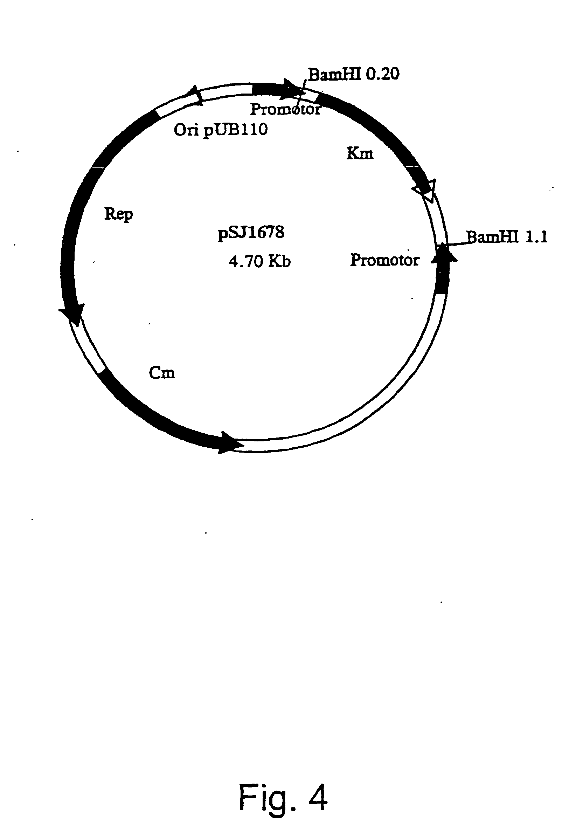

DNA was isolated from Bacillus sp. NCIMB 40916 by the method of Pitcher, D. G., Saunders,N. A., and Owen, R. J. (1989) Lett. Appl. Microbiol. 8, 151-156. Chromosomal DNA was partially digested with the restriction enzyme Sau3AI. The fragments were cloned into the BamHI site of the cloning vector pSJ1678, as shown in Figure 4 and transformed into Escherichia coli SJ2, thereby creating a gene library of Bacillus sp. NCIMB 40916.

This gene library was screened for alpha amylase activity and the strain showing alpha amylase activity was termed Escherichia coli strain NN049489 comprises the alpha-amylase of the invention. This strain was deposited and given the accession number DSM13001. Escherichia coli strain NN049489 harbouring the plasmid termed pJA386 encoding the alpha amylase was used for DNA sequencing.

### Example 2

### Sequencing of DNA and alpha-amylase

The alpha amylase gene cloned in plasmid pJA386 was characterized by DNA sequencing by primer walking, using the Taq deoxyterminal cycle sequencing kit (Perkin Elmer, USA), fluorescent labeled terminators and appropriate oligonucleotides as primers.

Analysis of the sequence data was performed according to Devereux et al. (1984) Nucleic Acids Res. 12, 387-395. The sequence corresponds to the DNA sequence shown in SEQ ID NO: 1. The predicted protein sequence of alpha amylase including the signal peptide and the mature alpha amylase are presented in SEQ ID NO: 2. The deduced N-terminal sequence was verified by sequencing 34 amino acids at the N-terminal of the protein.

### Example 3

### Production of alpha-amylase

*E. coli* NN049489 (DSM 13001) harboring plasmid pJA386 was cultivated over night in LB-broth containing chloramphenicol 10 *µ*g/ml, 37 °C, 250 rpm. Cells were harvested from 2.7 l culture broth by centrifugation at 6000 rpm for 15 minutes. The intracellular located amylase was released from the cells by using the following osmotic shock procedure:
1) Cells were resuspended and washed in 500 ml 10 mM Tris- HCl, pH 7.0 (EKV-buffer) followed by centrifugation.
2) Cells were resuspended in 75 ml 20% sucrose, 30 mM Tris-HCl pH 8, 1 mM EDTA and added Lysozyme to a concentration of 5 mg/ml.
3) The solution was incubated 15 min on ice followed by centrifugation. The majority of the amylase was now contained in the supernatant.

The supernatant was dialyzed overnight against 10 l in EKV-buffer with one buffer change to decrease the high concentration of sucrose. The solution was filtered through a 0.45 micro m membrane vacuum filter.

The enzyme solution was applied on a Pharmacia Q Sepharose column FF previously equilibrated in EKV-buffer, pH 7, and the column was washed with EKV-buffer. Bound proteins were eluted with a linear NaCl gradient from 0-500 mM over 10 column volumes. Amylase containing fractions were pooled and dialyzed against EKV-buffer over night.

The solution was then applied on a Q Sepharose column FF previously equilibrated in EKV-buffer, pH 8, the column was washed with EKV-buffer, and the amylase was eluted with a linear NaCl gradient from 0-500 mM over 10 column volumes. Amylase containing fractions were pooled.

A Phenyl Superose column previously equilibrated in EKV-buffer, pH 8 containing 1 M ammonium sulfate was loaded with the enzyme solution added ammonium sulfate to a concentration of 1 M. Unbound material was washed out with the ammonium sulfate buffer and the column was eluted with a linear NaCl gradient from 1-0 M ammonium sulfate over 20 column volumes. Amylase containing fractions were pooled.

The purified amylase was analyzed by SDS-PAGE and only one band was obtained after staining with Coomasie Blue.

### Example 4

### Washing test

Washing performance was evaluated by washing soiled test swatches for 15 minutes at 25°C in a detergent solution with the alpha-amylase of the invention.

The detergent used was the A/P Model Detergent described above at 3 g/l having pH 10.5, or a commercial detergent from Malaysia (FAB Total from Colgate) at 3 g/l having a pH of approx. 9.7. The purified amylase of Example 3 was added to the detergent solution at the concentration indicated below. The test swatches were soiled with orange rice starch (CS-28 swatches available from CFT, Center for Test Material, Holland).

After washing, the swatches were evaluated by measuring the remission at 460 nm. The results are expressed as △R = remission of the swatch washed with the alpha-amylase minus the remission of a swatch washed at the same conditions without the alpha-amylase.

| Alpha-amylase concentration (mg enzyme protein/l) | ΔR Model detergent | ΔR Malaysian detergent |
|---|---|---|
| 0 (reference) | = 0 | = 0 |
| 0.1 | 0.9 | 1 |
| 0.2 | 1.9 | 1.3 |
| 0.5 | 3 | 2.4 |
| 1.5 | 4.1 | 4.3 |

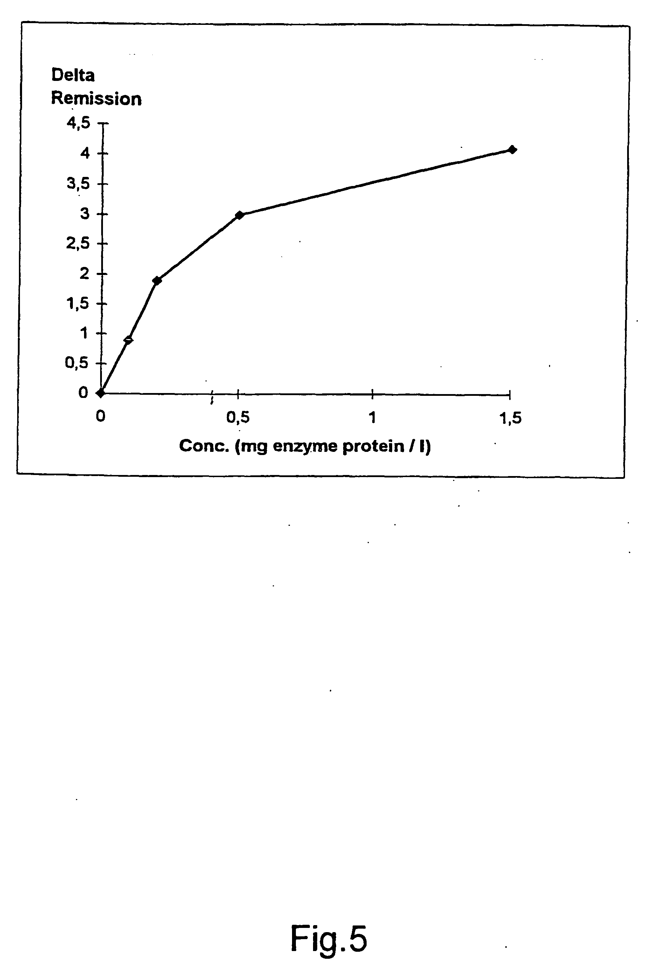

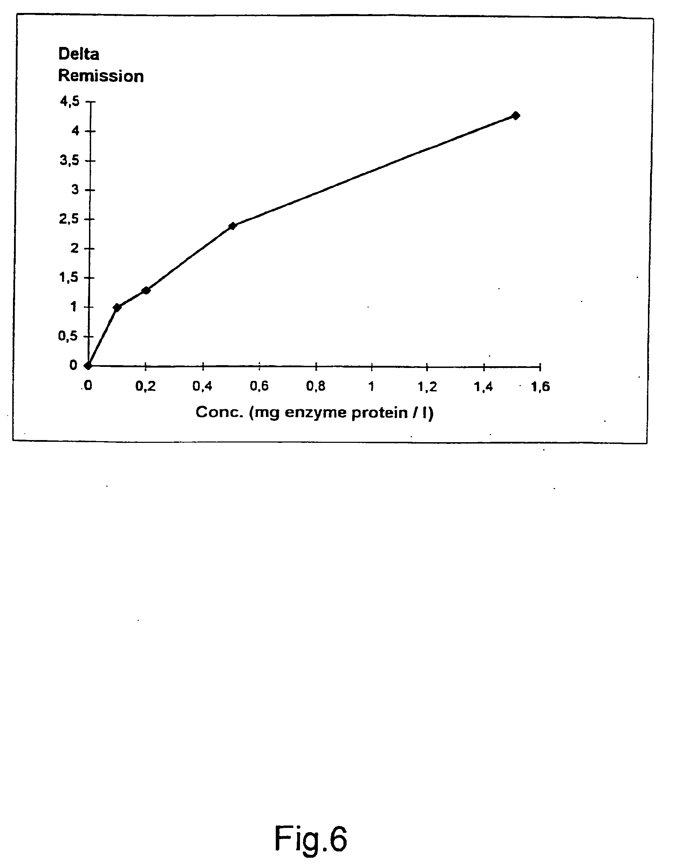

The results are shown in Figures 5 and 6. The results demonstrate that the alpha-amylase of the invention is effective in both detergents at highly alkaline pH.

## Claims

1. An alpha-amylase with improved performance in alkaline solutions which is:
a) a polypeptide having an amino acid sequence as shown in positions 1-556 of SEQ ID NO: 2, or
b) a polypeptide encoded by the alpha-amylase encoding part of the DNA sequence cloned into a plasmid present in *Escherichia coli* DSM 13001 (NN049489), or
c) a polypeptide which exhibits a degree of identity of at least 60% with the polypeptide defined in (a) or (b);
with the proviso that the polypeptide does not have the amino acid sequence as shown in positions 32-532 of SEQ ID NO:2 disclosed in WO 99/42567 or an analogue thereof which is at least 60% homologous with said polypeptide.

2. The alpha-amylase of claim 1, which has an activity at pH 10.5 which is at least two times higher than the activity at pH 7.3 when measured at 37°C.

3. The alpha-amylase of claim 1 or 2, which has an activity at pH 9.5 which is at least 4 times higher than the activity at pH 7.3 when measured at 37°C.

4. The alpha-amylase of any preceding claim, which has an optimum pH of about 9.5 when measured at 37°C.

5. The alpha-amylase of any preceding claim, which is from a strain of *Bacillus,* preferably *Bacillus* sp. NCIMB 40916.

6. The alpha-amylase of any preceding claim which retains more than 90 % of its activity after 20 minutes incubation at 25°C in a solution of 3 g/l of a test detergent containing 20% STPP, 25% Na₂SO₄, 15% Na₂CO₃, 20% LAS, 5% C12-C15 alcohol ethoxylate, 5% Na₂Si₂O₅, 0.3% NaCl at pH 10.5 and 6 degrees German hardness, and retains less than 90 % of its activity after 20 minutes incubation at 30°C in the same solution.

7. The alpha-amylase of any preceding claim, which has a molecular weight of about 55 kDa as determined by SDS-PAGE.

8. The alpha-amylase of any preceding claim, which has an isoelectric point of about 5 as determined by isoelectric focusing.

9. The alpha-amylase of any preceding claim in the form of a detergent additive which is a non-dusting granulate or a stabilized liquid.

10. An isolated DNA sequence which encodes the alpha-amylase of any of preceding claim.

11. The DNA sequence of claim 10, which comprises:
a) the DNA sequence shown in positions 94-1764 of SEQ ID NO:1, or
b) the alpha-amylase encoding part of the DNA sequence cloned into a plasmid present in *Escherichia coli* DSM 13001 (NN049489), or
c) a DNA sequence which has a degree of identity of at least 60% with the DNA sequence defined in a) or b);
with the proviso that the DNA sequence does not have the DNA sequence shown in positions 94-1596 of SEQ ID NO:1 disclosed in WO 99/42567 or an analogue thereof which is at least 60% homologous with said DNA sequence.

12. The DNA sequence according to claim 10 or 11, which is from a bacterium, preferably from *Bacillus,* most preferably from the strain NCIMB 40916.

13. A recombinant expression vector comprising the DNA sequence according to any of claims 10-12.

14. A cell transformed with the DNA sequence according to any of claims 10-12 or the recombinant expression vector according to claim 13.

15. The cell according to claim 14, which is a prokaryotic cell, in particular a bacterial cell or an endogenous cell from which said DNA sequence originates.

16. The cell according to claim 15, wherein the cell belongs to *Bacillus* or *Escherichia,* preferably *E*. *coli.*

17. A method of producing an alpha-amylase, comprising cultivating a cell according to any of claims 14-16 under conditions permitting the production of the alpha-amylase, and recovering the alpha-amylase from the culture.

18. A method for producing the alpha-amylase of any of claims 1-9, comprising cultivating an amylase-producing strain of Bacillus in a suitable nutrient medium, and recovering the alpha-amylase from the culture medium.

19. A detergent composition comprising the alpha-amylase of any of claims 1-9 and a surfactant.

20. The detergent composition of the preceding claim which has a pH of 8.5-11 in aqueous solution, preferably pH 9-10.5

21. The detergent composition of claim 19 or 20, which is a laundry detergent.

## Patentansprüche

1. Alpha-Amylase mit erhöhter Leistung in alkalischen Lösungen, die
a) ein Polypeptid mit einer Aminosäuresequenz wie in den Positionen 1-556 von SEQ ID Nr. 2 gezeigt, oder
b) ein Polypeptid, das durch den alpha-Amylase kodierenden Teil der DNA-Sequenz kodiert ist, die in ein in *Escherichia coli* DSM 13001 (NN049489) vorliegendes Plasmid geklont ist, oder
c) ein Polypeptid, das einen Identitätsgrad von mindestens 60% mit dem in (a) oder (b) definierten Polypeptid aufweist; ist
mit der Maßgabe, dass das Polypeptid nicht eine Aminosäuresequenz, wie in den Positionen 32-532 von SEQ ID Nr. 2 gezeigt, die in WO 99/42567 offenbart ist, oder ein Analog davon, das mindestens 60% homolog zu dem Polypeptid ist, besitzt.

2. Alpha-Amylase nach Anspruch 1, die eine Aktivität bei pH 10,5 aufweist, die mindestens zweimal höher ist als die Aktivität bei pH 7,3, wenn bei 37°C gemessen wird.

3. Alpha-Amylase nach Anspruch 1 oder 2, die eine Aktivität bei pH 9,5 aufweist, die mindestens viermal höher ist als die Aktivität bei pH 7,3, wenn bei 37°C gemessen wird.

4. Alpha-Amylase nach einem vorangehenden Anspruch, die ein pH Optimum von etwa 9,5 aufweist, wenn bei 37°C gemessen wird.

5. Alpha-Amylase nach einem vorangehenden Anspruch, die aus einem Stamm von *Bacillus,* vorzugsweise *Bacillus* sp. NCIMB 40916, ist.

6. Alpha-Amylase nach einem vorangehenden Anspruch, die mehr als 90% ihrer Aktivität nach 20 Minuten Inkubation bei 25°C in einer Lösung von 3 g/l eines Test-Waschmittels beibehält, enthaltend 20% STPP, 25% Na₂SO₄, 15% Na₂CO₃, 20% LAS, 5% C12-C15 Alkoholethoxylat, 5% Na₂Si₂O₅, 0,3 % NaCl bei pH 10,5 und Deutscher Härtegrad 6 und weniger als 90% ihrer Aktivität nach 20 Minuten Inkubation bei 30°C in der gleichen Lösung beibehält.

7. Alpha-Amylase nach einem vorangehenden Anspruch, die ein Molekulargewicht von etwa 55 kDa aufweist, wie durch SDS-PAGE bestimmt.

8. Alpha-Amylase nach einem vorangehenden Anspruch, die einen isoelektrischen Punkt von etwa 5 aufweist, wie durch Isoelektrische Fokussierung bestimmt.

9. Alpha-Amylase nach einem vorangehenden Anspruch, in der Form eines Waschmittelzusatzes, dass ein nicht staubendes Granulat oder eine stabilisierte Flüssigkeit ist.

10. Isolierte DNA-Sequenz, die die alpha-Amylase nach einem vorangehenden Anspruch kodiert.

11. DNA-Sequenz nach Anspruch 10, die:
a) eine DNA-Sequenz in den Positionen 94-1764 von SEQ ID Nr. 1 gezeigt, oder
b) eine alpha-Amylase, die einen kodierenden Teil der DNA-Sequenz, die in ein in *Escherichia coli* DSM 13001 (NN049489) vorliegendes Plasmid geklont ist, oder
c) eine DNA-Sequenz, die einen Identitätsgrad von mindestens 60% mit der in (a) oder (b) bestimmten DNA-Sequenz aufweist; umfasst
mit der Maßgabe, dass die DNA-Sequenz nicht eine DNA-Sequenz, in den Positionen 94-1596 von SEQ ID Nr. 1 gezeigt, die in WO 99/42567 offenbart ist, oder ein Analog davon, das mindestens 60 % homolog zu der DNA-Sequenz ist, besitzt.

12. DNA-Sequenz nach Anspruch 10 oder 11, die von einem Bakterium, vorzugsweise von *Bacillus,* besonders bevorzugt aus dem Stamm NCIMB 40916 ist.

13. Rekombinanter Expressionsvektor, umfassend eine DNA-Sequenz nach einem der Ansprüche 10-12.

14. Zelle, die mit der DNA-Sequenz nach einem der Ansprüche 10-12 oder dem rekombinanten Expressionsvektor nach Anspruch 13 transformiert ist.

15. Zelle nach Anspruch 14, die eine prokaryontische Zelle ist, insbesondere eine Bakterienzelle oder eine endogene Zelle, von der die DNA-Sequenz stammt.

16. Zelle nach Anspruch 15, wobei die Zelle zu *Bacillus* oder *Escherichia,* vorzugsweise *E. coli,* gehört.

17. Verfahren zur Herstellung einer alpha-Amylase, umfassend das Kultivieren einer Zelle nach einem der Ansprüche 14-16, unter Bedingungen, die die Herstellung der alpha-Amylase erlauben, und Gewinnen der alpha-Amylase aus der Kultur.

18. Verfahren zur Herstellung einer alpha-Amylase nach einem der Ansprüche 1-9, umfassend das Kultivieren eines amylaseherstellenden Stamms von Bacillus in einem geeigneten Nährstoffmedium und Gewinnen der alpha-Amylase aus der Kultur.

19. Waschmittelzusammensetzung, umfassend eine alpha-Amylase nach einem der Ansprüche 1-9 und ein Tensid.

20. Waschmittelzusammensetzung nach dem vorangehenden Anspruch, die einen pH von 8,5-11 in wässriger Lösung aufweist, vorzugsweise pH 9-10,5.

21. Waschmittelzusammensetzung nach Anspruch 19 oder 20, die ein Haushaltswaschmittel ist.

## Revendications

1. Alpha-amylase ayant des performances améliorées dans des solutions alcalines, qui est :
a) un polypeptide ayant une séquence d'acides aminés telle que représentée aux positions 1 à 556 de SEQ ID N° 2, ou
b) un polypeptide codé par la partie codant une alpha-amylase de la séquence d'ADN clonée dans un plasmide présent dans *Escherichia coli* DSM 13001 (NN049489), ou
c) un polypeptide qui présente un degré d'identité d'au moins 60% avec le polypeptide défini en (a) ou (b) ;
à la condition que le polypeptide n'ait pas la séquence d'acides aminés telle que représentée aux positions 32 à 532 de SEQ ID N° 2 divulguée dans WO 99/42567 ou d'un analogue de celle-ci qui est homologue à au moins 60% audit polypeptide.

2. Alpha-amylase selon la revendication 1, qui a une activité à pH 10,5 qui est au moins deux fois supérieure à l'activité à pH 7,3 mesurée à 37°C.

3. Alpha-amylase selon la revendication 1 ou 2, qui a une activité à pH 9,5 qui est au moins 4 fois supérieure à l'activité à pH 7,3 mesurée à 37°C.

4. Alpha-amylase selon l'une quelconque des revendications précédentes, qui a un pH optimal d'environ 9,5 mesuré à 37°C.

5. Alpha-amylase selon l'une quelconque des revendications précédentes, qui provient d'une souche de *Bacillus,* de préférence *Bacillus* sp. NCIMB 40916.

6. Alpha-amylase selon l'une quelconque des revendications précédentes, qui conserve plus de 90% de son activité après incubation pendant 20 minutes à 25°C dans une solution de 3 g/l d'un détergent de test contenant 20% de STPP, 25% de Na₂SO₄, 15% de Na₂CO₃, 20% de LAS, 5% d'éthoxylate d'alcool en C₁₂ à C₁₅, 5% de Na₂Si₂O₅, 0,3% de NaCl à pH 10,5 et une dureté German de 6 degrés, et qui conserve moins de 90% de son activité après incubation pendant 20 minutes à 30°C dans la même solution.

7. Alpha-amylase selon l'une quelconque des revendications précédentes, qui a une masse moléculaire d'environ 55 kDa telle que déterminée par SDS-PAGE.

8. Alpha-amylase selon l'une quelconque des revendications précédentes, qui a un point isoélectrique d'environ 5 tel que déterminé par focalisation isoélectrique.

9. Alpha-amylase selon l'une quelconque des revendications précédentes, sous la forme d'un additif pour détergent qui est un granulé non poussiéreux ou un liquide stabilisé.

10. Séquence d'ADN isolée qui code l'alpha-amylase selon l'une quelconque des revendications précédentes.

11. Séquence d'ADN selon la revendication 10, qui comprend :
a) la séquence d'ADN représentée aux positions 94 à 1764 de SEQ ID N° 1, ou
b) la partie codant une alpha-amylase de la séquence d'ADN clonée dans un plasmide présent dans *Escherichia coli* DSM 13001 (NN049489), ou
c) une séquence d'ADN qui a un degré d'identité d'au moins 60% avec la séquence d'ADN définie en a) ou b) ;
à la condition que la séquence d'ADN n'ait pas la séquence d'ADN représentée aux positions 94 à 1596 de SEQ ID N° 1 présentée dans WO 99/42567 ou d'un analogue de celle-ci qui est homologue à au moins 60% à ladite séquence d'ADN.

12. Séquence d'ADN selon la revendication 10 ou 11, qui est issue d'une bactérie, de préférence de *Bacillus,* de la façon la plus préférentielle de la souche NCIMB 40916.

13. Vecteur d'expression recombinant comprenant la séquence d'ADN selon l'une quelconque des revendications 10 à 12.

14. Cellule transformée avec la séquence d'ADN selon l'une quelconque des revendications 10 à 12 ou avec le vecteur d'expression recombinant selon la revendication 13.

15. Cellule selon la revendication 14, qui est une cellule procaryote, en particulier une cellule bactérienne ou une cellule endogène à partir de laquelle ladite séquence d'ADN est issue.

16. Cellule selon la revendication 15, dans laquelle la cellule appartient au genre *Bacillus* ou *Escherichia,* de préférence *E. coli.*

17. Procédé de production d'une alpha-amylase, comprenant la culture d'une cellule selon l'une quelconque des revendications 14 à 16, dans des conditions qui permettent la production de l'alpha-amylase, et la récupération de l'alpha-amylase à partir de la culture.

18. Procédé de production de l'alpha-amylase selon l'une quelconque des revendications 1 à 9, comprenant la culture d'une souche de *Bacillus* produisant une amylase dans un milieu nutritif adéquat et la récupération de l'alpha-amylase à partir du milieu de culture.

19. Composition de détergent comprenant l'alpha-amylase selon l'une quelconque des revendications 1 à 9 et un agent tensioactif.

20. Composition de détergent selon la revendication précédente, qui a un pH de 8,5 à 11 en solution aqueuse, de préférence un pH de 9 à 10,5.

21. Composition de détergent selon la revendication 19 ou 20, qui est un détergent pour le linge.
